# EUROPÄISCHE PATENTSCHRIFT

(11) **EP 2 848 911 B1**
(45) Veröffentlichungstag und Bekanntmachung des Hinweises auf die Patenterteilung: **19.10.2016**
(21) Anmeldenummer: 14183055.4
(22) Anmeldetag: 01.09.2014
(51) Int. Cl.: A61B 90/00, G01M 5/00

(54) **Testvorrichtung mit kardanisch aufgehängter Halterung für Katheter**
Test apparatus with gimbaled mount for catheters
Dispositif de test doté d'un support suspendu à la cardan pour cathéter

(30) Priorität: 12.09.2013 US 201361876771 P
(43) Veröffentlichungstag der Anmeldung: 18.03.2015
(73) Patentinhaber: BIOTRONIK SE & Co. KG, 12359 Berlin (DE)
(72) Erfinder: Feldmann, Jörg, 12683 Berlin (DE); Widdecke, Heinrich, 14476 Potsam (DE); Kolberg, Gernot, 12161 Berlin (DE)
(74) Vertreter: Galander, Marcus

(56) Entgegenhaltungen:
- GB-A- 2 264 177
- GB-A- 2 361 774
- US-A1- 2008 257 061

## Beschreibung

Die Erfindung betrifft eine Testvorrichtung zur Durchführung von Stauchtests an Kathetern, mit einer eine Aufnahmeöffnung für ein Ende des Katheters aufweisenden Halterung. Bei der Durchführung von Stauchtests werden Katheter mit einem Ende und beispielsweise mit ihrer Spitze in die Aufnahmeöffnung eingesetzt und zum Stauchen in diese hineingedrückt. Dabei biegen sich die Katheter seitlich undefiniert weg und liegen hierdurch undefiniert in der Aufnahmeöffnung, wodurch das Ergebnis des Stauchtests beeinflusst wird. Ferner kann der Katheter aus der Aufnahmeöffnung herausrutschen, wodurch der Katheter und insbesondere eine Elektrodenspitze von dessen Schraubelektrode beschädigt werden kann. Wird die Spitze fixiert, beispielsweise durch Einschrauben der Schraubelektrode in eine durch die Fixierhelix punktierbare Masse, so kann die Elektrodenspitze und insbesondere ein gegebenenfalls polierter Anschliff der Elektrodenspitze beschädigt werden.

Die Patentanmeldung GB 2 361 774 A offenbart eine kardanische Klemme in einer Testapparatur für Biegungsversteifer, wie sie an Steigrohren in der Offshore-Öl- und - Gasindustrie verwendet werden.

Die US 2008/0257061 A1 beschreibt eine Testapparatur für die Stentbefestigungstechnik auf Ballon-Kathetern. Das Dokument beschäftigt sich mit der Bestimmung von Zug- bzw. Dehnkräften, die nötig wären, um einen gekrimpten Stent von einem Ballon und einem Katheter zu entfernen. Als Testvorrichtung für Katheter ist diese Vorrichtung jedoch nicht geeignet.

Das Dokument "Buckling Test as a New Approach to Testing Fexural Rigidities of Angiographic Catheters" von J. Carey et al. vom 21.01.2005, offenbart einen gebräuchlichen Kathetertest.

Der Erfindung liegt daher die Aufgabe zugrunde, eine Testvorrichtung bereitzustellen, mit der Stauchtests an Kathetern effizient und zweckmäßig durchgeführt werden können, ohne einerseits den zu testenden Katheter zu beschädigen und andererseits eine dem durch den Katheter anhaftendes individuelles Krümmungsverhalten in alle Richtungen frei kippbare Halterung zu verleihen.

Für die eingangs genannte Testvorrichtung ist die Aufgabe durch den Gegenstand des Anspruchs 1 gelöst.

Dadurch, dass die Aufhängung um zwei Achsen drehbar gelagert ist, kann sie jeder beliebigen seitlichen Biegung des Katheters während des Stauchtests folgen und die Katheterspitze auch bei größeren seitlichen Ausweichbewegungen des Katheters sicher halten. Ein Rutschen der Katheterspitze aus der Aufnahmeöffnung heraus ist hierdurch sicher verhindert. Während des Stauchtests ist die mögliche Bewegung des Katheters also auf Biegungen um einen vorbestimmten und durch die Drehachsen bestimmten Schwenkpunkt beschränkt. Durch die frei bewegliche Aufnahmeöffnung kann sich die Elektrode je nach ihrer Stabilität ungehindert krümmen. Somit werden durch diese Aufhängung weitestgehend die realen Bedingungen im Herzen nachvollzogen.

Die Zweckmäßigkeit liegt im Folgenden begründet:
1. Es besteht eine Halterung für die Katheterspitze, die horizontal zur vertikalen Schubrichtung des Katheters in alle Richtungen kippbar ist.
2. Durch diese technische Voraussetzung passt sich während des Vorschubs des Katheters die Ebene der Anschlagfläche der Halterung 100%ig dem spezifischen Krümmungsverhalten des Katheters an, wobei während des Vorschubs durch die freie Beweglichkeit der Halterung keine Querkräfte entstehen können.

Die erfindungsgemäße Lösung kann durch verschiedene, jeweils für sich vorteilhafte und, sofern nicht anders ausgeführt, beliebig miteinander kombinierbare Ausgestaltung weiter verbessert werden. Auf diese Ausgestaltungsformen und die mit ihnen verbundenen Vorteile ist im Folgenden eingegangen.

So kann eine Längsachse der Aufnahmeöffnung, die durch eine Bohrung ausgeformt sein kann, senkrecht zu den Drehachsen verlaufen. In einer vorbestimmten Schwenkposition der Halterung kann deren Längsachse sogar zu beiden Drehachsen senkrecht verlaufen.

Entlang der Längsachse, die sich vorzugsweise zentral durch die Aufnahmeöffnung erstreckt, kann die Katheterspitze in die Aufnahmeöffnung eingesetzt werden. Dadurch, dass die Längsachse senkrecht zu den Drehachsen verläuft, schwenkt die Katheterspitze bei Durchführung des Stauchtests ohne eine Vorzugsrichtung um die Drehachsen.

Um zusätzlich zu verhindern, dass sich die Halterung bei der Durchführung von Stauchtests in einer Vorzugsrichtung verdreht, kann die Längsachse die beiden Drehachsen schneiden.

Zur Vermeidung von ungewünschten auf die Halterung beziehungsweise auf die Katheterspitze wirkenden Kräften, schneiden die Drehachsen die Anschlagfläche der Halterung.

Die Aufnahmeöffnung kann gestuft ausgebildet sein. Beispielsweise kann die Aufnahmeöffnung quer zu ihrer Längsachse in einem ersten Bereich einen größeren Durchmesser aufweisen, als in einem zweiten Bereich, der sich entlang der Längsachse an den ersten Bereich anschließt. Die gestufte Aufnahmeöffnung kann also eine Stufe aufweisen, die die Längsachse zumindest teilweise oder sogar vollständig umlaufen kann. Eine Fläche der Stufe, die entgegen einer Einsetzrichtung, in der der Katheter in die Aufnahmeöffnung einsetzbar ist, weist, stellt eine Anschlagfläche für den Katheter bereit. Der Katheter kann demnach mit einer definierten Tiefe in die Aufnahmeöffnung eingesetzt werden. An der Anschlagfläche kann beispielsweise ein Mantel des Katheters anliegen, der durch den Test nicht beschädigt wird. Die Anschlagfläche ist vorzugsweise senkrecht zur Längsachse ausgebildet. In der Einsetzrichtung hinter der Anschlagfläche kann sich der zweite Bereich der Aufnahmeöffnung erstrecken, in den beim Stauchtest die Katheterspitze ragen kann. Ein direkter Kontakt der Katheterspitze mit der Halterung kann also vermieden werden. Folglich wird die Katheterspitze während des Stauchtests nicht beschädigt.

Um zu vermeiden, dass die Bewegung der Halterung während des Stauchtest eine Komponente in eine Vorzugsrichtung aufweist, können die Drehachsen die Stufe und insbesondere die Anschlagfläche schneiden. Entlang der Längsachse lastet der Katheter also auf der Stufe und insbesondere auf der durch die Stufe bereitgestellten Anschlagfläche, wobei die Stufe beziehungsweise die Anschlagfläche in einer Ebene mit den Drehachsen angeordnet sein können. Würde die Stufe beziehungsweise die Anschlagfläche entlang der Längsachse vor oder hinter den Drehachsen angeordnet sein, so würde ein durch den Katheter auf die Stufe oder die Anschlagfläche ausgeübter Druck ungewünschte Drehbewegungen der Halterung hervorrufen.

Damit die Testvorrichtung flexibel und mit unterschiedlich dimensionierten Kathetern verwendet werden kann, kann die Halterung ein Einsetzelement aufweisen, durch das sich die Aufnahmeöffnung zumindest teilweise oder sogar vollständig erstreckt und das wiederholt an einem Grundkörper der Halterung anbringbar und von diesem entfernbar, somit also auswechselbar ist. Zum Beispiel kann das Einsetzelement in eine Öffnung des Grundkörpers einsetzbar und aus dieser entnehmbar sein. Insbesondere wenn das Einsetzelement in die Aufnahmeöffnung eingesetzt ist, können die Drehachsen durch die Ebene der Anschlagfläche der Halterung verlaufen und/oder die Stufe beziehungsweise die Anschlagfläche schneiden.

Beispielsweise kann die Testvorrichtung zwei oder mehr Einsetzelemente mit unterschiedlich dimensionierten Aufnahmeöffnungen aufweisen, wobei die Einsetzelemente gegeneinander tauschbar mit dem Grundkörper verbindbar sind. Vorzugsweise weisen die Aufnahmeöffnungen und insbesondere der ersten und/oder der zweite Bereich der Aufnahmeöffnungen unterschiedliche Durchmesser auf. Entlang der Längsachse ist die Stufe bevorzugt bei allen Einsetzelementen gleich positioniert, sodass bei Verwendung eines beliebigen der Einsetzelemente keine ungewünschten Kräfte oder Bewegungen mit Vorzugsrichtungen hervorgerufen werden, welche die Bewegung Halterung in einer ungewollten und unzweckmäßigen Art und Weise beeinflussen.

Im Folgenden ist die Erfindung beispielhaft anhand einer Ausführungsform mit Bezug auf die Zeichnung erläutert. Die unterschiedlichen Merkmale der Ausführungsform können dabei unabhängig voneinander kombiniert werden, wie es bei den einzelnen vorteilhaften Ausgestaltungen bereits dargelegt wurde. Es zeigt:

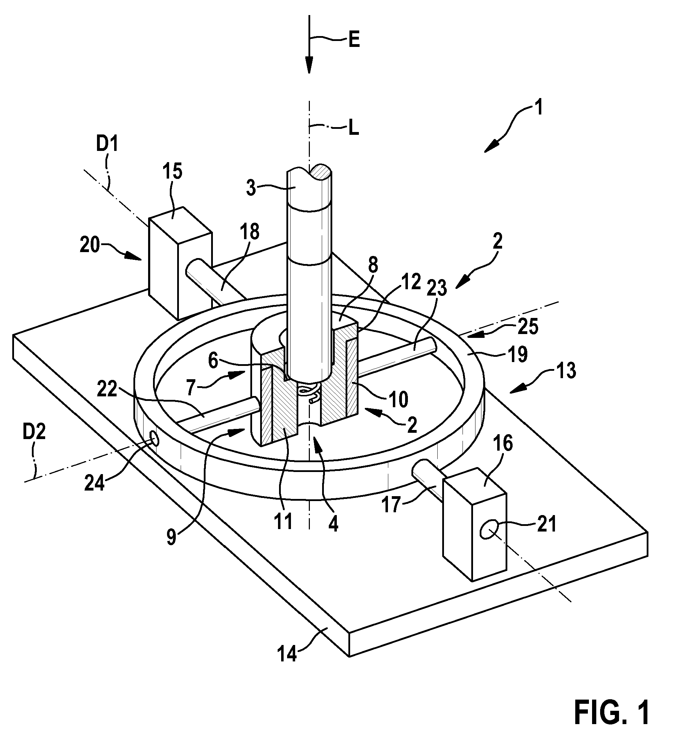

- Fig. 1: eine schematische Darstellung eines ersten Ausführungsbeispiel der erfindungsgemäßen Testvorrichtung.

Aufbau und Funktion einer erfindungsgemäßen Testvorrichtung ist im Folgenden mit Bezug auf das Ausführungsbeispiel der Figur 1 beschrieben:

Figur 1 zeigt die Testvorrichtung 1 zur Durchführung von Stauchtests schematisch in einer perspektivischen Ansicht. Die Testvorrichtung 1 weist eine Halterung 2 für ein Ende eines zu testenden Katheters 3 auf. Die Halterung 2 ist mit einer Aufnahmeöffnung 4 ausgebildet, in die eine Spitze des Katheters 3 einsetzbar ist.

Die Aufnahmeöffnung 4 erstreckt sich entlang ihrer Längsachse L durch die Halterung 2 und ist beispielsweise mit einem kreisförmigen Querschnitt ausgebildet. Entlang der Längsachse L verjüngt sich ein Innendurchmesser der Aufnahmeöffnung 4 sprungartig, sodass die Aufnahmeöffnung 4 gestuft ausgebildet ist. Die durch die gestufte Ausformung der Aufnahmeöffnung 4 ausgebildete Stufe 5 stellt eine Anschlagfläche 6 für den Katheter 3 und insbesondere für einen Mantel des Katheters 3 bereit. Die Anschlagfläche 6 ist vorzugsweise horizontal zur Längsachse L ausgebildet und umläuft die Aufnahmeöffnung 4 um die Längsachse L herum.

In einer parallel zur Längsachse L verlaufenden Einsetzrichtung E ist der Katheter 3 mit seiner Spitze in die Aufnahmeöffnung 4 einsetzbar. Dabei wird der Katheter 3 zunächst in einen ersten Bereich 7 der Aufnahmeöffnung 4 eingesetzt. Der erste Bereich 7 erstreckt sich entgegen der Einsetzrichtung E von der Stufe 5 bis zu einem Längsende 8 der Halterung 2. In der Einsetzrichtung E schließt sich an die Stufe 5 und/oder an den ersten Bereich 7 ein zweiter Bereich 9 der Aufnahmeöffnung 4 an, wobei ein Innendurchmesser des zweiten Bereichs 9 kleiner ist, als ein Innendurchmesser des ersten Bereichs 7.

Optional kann die Halterung 2 mehrteilig und insbesondere zweiteilig ausgebildet sein. Beispielsweise kann die Halterung 2 einen Grundkörper 10 aufweisen, der beispielsweise als eine sich entlang der Längsachse L erstreckende Hülse ausgeformt sein kann. Ferner kann die Halterung 2 ein Einsetzelement 11 aufweisen, das am Grundkörper 10 befestigt ist beziehungsweise befestigbar sein kann. Ist der Grundkörper 10 als eine Hülse ausgebildet, kann das Einsetzelement 11 in den Grundkörper 10 eingesetzt sein.

Das Einsetzelement 11 kann die Aufnahmeöffnung 4 und optional auch die Stufe 5 beziehungsweise die durch die Stufe 5 bereitgestellte Anschlagfläche 6 aufweisen. Ist das Einsetzelement 11 am Grundkörper 10 angebracht beziehungsweise in diesen eingesetzt, bildet das Einsetzelement 11 vorzugsweise das Längsende 8 der Halterung 2. Dabei kann das Einsetzelement 11 einen quer zur Längsachse L und von der Längsachse L weg verlaufenden Vorsprung 12 aufweisen, der ein zu tiefes Einsetzen des Einsetzelementes 11 in den Grundkörper 10 verhindert. Der Vorsprung 12 kann sich vollständig um die Längsachse L herum erstrecken.

Im Ausführungsbeispiel der Figur 1 ist die Aufnahmeöffnung 4 als eine durchgehende Öffnung ausgeformt. Alternativ kann die Aufnahmeöffnung 4 und insbesondere der zweite Bereich 9 der Aufnahmeöffnung 4 in der Einsetzrichtung E jedoch auch geschlossen ausgeformt sein. Die Aufnahmeöffnung 4 ist jedoch bevorzugt eine durchgängige Öffnung, um zu verhindern, dass die Katheterspitze mit einem Grund der Aufnahmeöffnung 4 oder mit sich am Grund der Aufnahmeöffnung 4 ansammelnden Verunreinigungen kollidiert.

Um zu vermeiden, dass sich die Katheterspitze während des Tests ungewünscht relativ zur Halterung 2 bewegt, weist die Testvorrichtung 1 eine kardanische Aufhängung 13 auf, in der die Halterung 2 um zwei Drehachsen D1, D2 herum drehbar aufgehängt ist. Die Katheterspitze des Katheters 3 kann also zusammen mit der Halterung 2 um die Drehachsen D1, D2 herum schwenken, sodass beim Stauchtest auftretende Bewegungen des Katheters 3 nicht zu Relativbewegungen zwischen der Katheterspitze und der Halterung 2 sowie zu einem Herausrutschen der Katheterspitze aus der Halterung 2 führen können.

Die kardanische Aufhängung 13 weist beispielsweise zwei auf einer Grundplatte 14 der Testvorrichtung 1 angeordnete Lagereinrichtungen 15, 16 auf. Die Lagereinrichtungen 15, 16 sind vorzugsweise unverschieblich an der Grundplatte 14 angebracht. An den Lagereinrichtungen 15, 16 sind zum Beispiel Stangen beziehungsweise Stäbe 17, 18 angebracht, welche jeweils eine der Lagereinrichtungen 15, 16 mit einem Lagerring 19 der kardanischen Aufhängung 13 verbinden. Durch die Stangen 17, 18 erstreckt sich die Drehachse D1, sodass der Lagerring 19 um die Drehachse D1 herum drehbar ist. Dabei kann der Lagerring 19 Lagerelemente aufweisen, welche ein Drehen des Lagerrings 19 relativ zu den Stangen 17, 18 ermöglichen. Alternativ und wie zum Ausführungsbeispiel der Figur 1 dargestellt, weisen die Lagereinrichtungen 15, 16 jeweils ein Lagerelement 20, 21 auf, an dem die Stangen 17, 18 um die Drehachse D1 herum drehbar gelagert sind. Beispielsweise sind die Lagerelemente 20, 21 Kugellager, die ein reibungsarmes Verdrehen der Stangen 17, 18 um die Drehachse D1 herum ermöglichen. Am Lagerring 19 können die Stangen 17, 18 unbeweglich befestigt sein.

Die Halterung 2 ist vorzugsweise über zwei Stangen 22, 23 mit dem Lagerring 19 drehbar verbunden. Dabei erstrecken sich die Stangen 22, 23 vorzugsweise entlang der Drehachse D2, sodass die Halterung 2 relativ zum Lagerring 19 um die Drehachse D2 herum drehbar ist. Die Halterung 2 kann Lagerelemente aufweisen, welche ein Verdrehen der Halterung 2 um die Drehachse D2 und relativ zu den Stangen 22, 23 herum ermöglichen. Vorzugsweise und wie im Ausführungsbeispiel der Figur 1 dargestellt, sind die Lagerelemente 24, 25 jedoch am Lagerring 19 vorgesehen, sodass die Stangen 22, 23 relativ zum Lagerring 19 um die Drehachse D2 herum drehbar sind. An der Halterung 2 und insbesondere an deren Grundkörper 10 können die Stangen 22, 23 unbeweglich befestigt sein.

## Patentansprüche

1. Testvorrichtung (1) zur Durchführung von Stauchtests an Kathetern (3), mit einer eine Aufnahmeöffnung (4) für ein Ende des Katheters (3) aufweisenden Halterung (2), wobei die Halterung eine kardanische Aufhängung (13) aufweist, in der die Halterung (2) um zwei Drehachsen (D1, D2) drehbar gelagert ist, und wobei die Aufnahmeöffnung eine Anschlagfläche (6) aufweist, **dadurch gekennzeichnet, dass** die Drehachsen (D1, D2) die Anschlagfläche der Halterung (2) schneiden.

2. Testvorrichtung (1) nach Anspruch 1, **dadurch gekennzeichnet, dass** eine Längsachse (L) der Aufnahmeöffnung (4) senkrecht zu einer der Drehachsen (D2) verläuft.

3. Testvorrichtung (1) nach Anspruch 2, **dadurch gekennzeichnet, dass** die Längsachse (L) die Drehachsen (D1, D2) schneidet.

4. Testvorrichtung (1) nach einem der Ansprüche 1 bis 3, **dadurch gekennzeichnet, dass** die Aufnahmeöffnung (4) gestuft ausgebildet ist.

5. Testvorrichtung (1) nach Anspruch 4, **dadurch gekennzeichnet, dass** die Drehachsen (D1, D2) eine Stufe (5) der gestuften Aufnahmeöffnung (4) schneiden.

6. Testvorrichtung (1) nach einem der Ansprüche 1 bis 5, **dadurch gekennzeichnet, dass** die Halterung (2) ein Einsetzelement (11) aufweist, durch das sich die Aufnahmeöffnung (4) zumindest teilweise erstreckt, und das wiederholt an einem Grundkörper (10) der Halterung (2) anbringbar und von diesem entfernbar ist.

7. Testvorrichtung (1) nach Anspruch 6, **dadurch gekennzeichnet, dass** die Testvorrichtung (1) zwei Einsetzelemente (11) mit unterschiedlich dimensionierten Aufnahmeöffnungen (4) aufweist, wobei die Einsetzelemente (11) gegeneinander tauschbar mit dem Grundkörper (10) verbindbar sind.

## Claims

1. A test apparatus (1) for performing compression tests on catheters (3), comprising a mount (2) having a receiving opening (4) for an end of the catheter (3), wherein the mount has a gimbal (13), in which the mount (2) is mounted rotatably about two axes of rotation (D1, D2), and wherein the receiving opening has a stop face (6), **characterised in that** the axes of rotation (D1, D2) cross the stop face of the mount (2).

2. The test apparatus (1) according to claim 1, **characterised in that** a longitudinal axis (L) of the receiving opening (4) runs perpendicularly to one of the axes of rotation (D2).

3. The test apparatus (1) according to claim 2, **characterised in that** the longitudinal axis (L) crosses the axes of rotation (D1, D2).

4. The test apparatus (1) according to any one of claims 1 to 3, **characterised in that** the receiving opening (4) is stepped.

5. The test apparatus (1) according to claim 4, **characterised in that** the axes of rotation (D1, D2) cross a step (5) of the stepped receiving opening (4).

6. The test apparatus (1) according to any one of claims 1 to 5, **characterised in that** the mount (2) has an insert element (11), through which the receiving opening (4) extends at least in part and which can be repeatedly attached to and removed from a main body (10) of the mount (2).

7. The test apparatus (1) according to claim 6, **characterised in that** the test apparatus (1) has two insert elements (11) with receiving openings (4) of different dimension, wherein the insert elements (11) can be connected to the main body (10) in a manner exchangeable for one another.

## Revendications

1. Dispositif de test (1) pour la réalisation de tests de compression sur des cathéters (3), avec un orifice d'admission (4) pour un support (2) présentant une extrémité du cathéter (3), où le support présente une suspension à cardan (13), dans laquelle le support (2) est logé en pouvant être mis en rotation autour de deux axes de rotation (D1, D2), et où l'orifice d'admission présente une surface d'appui (6), **caractérisé en ce que** les axes de rotation (D1, D2) coupent la surface d'appui du support (2).

2. Dispositif de test (1) selon la revendication 1, **caractérisé en ce qu'**un axe longitudinal (L) de l'orifice d'admission (4) s'étend verticalement par rapport à l'un des axes de rotation (D2).

3. Dispositif de test (1) selon la revendication 2, **caractérisé en ce qu'**un axe longitudinal (L) coupe les axes de rotation (D1, D2).

4. Dispositif de test (1) selon l'une des revendications 1 à 3, **caractérisé en ce que** l'orifice d'admission (4) est conçu par paliers.

5. Dispositif de test (1) selon la revendication 4, **caractérisé en ce que** les axes de rotation (D1, D2) coupent un palier (5) de l'orifice d'admission (4) en paliers.

6. Dispositif de test (1) selon l'une des revendications 1 à 5, **caractérisé en ce que** le support (2) présente un élément d'insertion (11), par lequel l'orifice d'admission (4) peut être en extension au moins partiellement, et qui peut être rapporté sur un corps de base (10) du support (2) et en être enlevé de manière répétée.

7. Dispositif de test (1) selon la revendication 6, **caractérisé en ce que** le dispositif de test (1) présente deux éléments d'insertion (11) avec des orifices d'admission (4) dimensionnés de manière différente, où les éléments d'insertion (11) peuvent être reliés au corps de base (10) en pouvant être échangés mutuellement.
